# EUROPEAN PATENT APPLICATION

(11) **EP 2 236 128 A1**
(43) Date of publication of application: **06.10.2010**
(21) Application number: 08863296.3
(22) Date of filing: 17.12.2008
(51) Int. Cl.: A61K 8/891, A61K 8/31, A61K 8/60, A61K 8/73, A61Q 1/10

(54) **COSMETIC FOR EYELASHES**

(30) Priority: 18.12.2007 JP 2007326096
(71) Applicant: Shiseido Company, Ltd., Chuo-ku Tokyo 104-8010 (JP)
(72) Inventor: IDE, Nobuyuki, Yokohama-shi Kanagawa 224-8558 (JP); TATSUTA, Amane, Yokohama-shi Kanagawa 224-8558 (JP); SATO, Fumitaka, Yokohama-shi Kanagawa 224-8558 (JP)
(74) Representative: Gendron, Vincent Christian
(86) International application number: PCT/JP2008/073016
(87) International publication number: WO 2009/078451

(57) **Abstract**

Object To provide a cosmetic for eyelashes, which is excellent in the effect of imparting a feeling of voluminousness to eyelashes and exerts high base transparency without damage to the color inherent in the cosmetic base.

Means of attaining the object A cosmetic for eyelashes which comprises (a) a silicone resin powder of particles having such a spiky-surface-sugar-candy-ball-like shape that small protrusions are present over the whole surface of each particle, (b) a volatile oil (e.g., low-boiling-point isoparaffinic hydrocarbon oil, low-boiling-point silicone oil, etc.), and (c) an oily gelling agent. Preferably, the cosmetic contains 1 - 20% by weight of component (a), 10 - 80% by mass of component (b) and 1 - 25% by mass of component (c). The cosmetic may optionally contain (d) a film-forming agent.

## Description

### TECHNICAL FIELD

The present invention relates to a cosmetic for eyelashes. More precisely, the invention relates to an oil-based cosmetic for eyelashes which is excellent in the effect of imparting a feeling of voluminousness to eyelashes and which exerts high base transparency without damage to the color inherent in the cosmetic base.

### BACKGROUND ART

Cosmetics for eyelashes such as typically mascara and the like are required to have the effect of making eyelashes look dense and long and the effect of imparting a feeling of voluminousness to eyelashes, and are additionally required to exert high base transparency without damage to the color (e.g., blackness) inherent in the cosmetic base.

As a means of imparting a feeling of voluminousness to eyelashes, there is known a technique of incorporating a powder in a base material; and for example, JP 2003-246710 A (Patent Reference 1) describes a cosmetic for eyelashes that contains degradable spherical boron nitride particles comprising a platelike boron nitride having a specific plate diameter and an inorganic powder having a specific particle diameter, and an oil-soluble resin; and JP 2004-315420 A (Patent Reference 2) describes mascara that contains a nonaqueous polymer dispersion with a polymer dispersed in a volatile silicone, and hollow particles.

However, in general, when a powder is incorporated in a base material and when the mascara is applied to eyelashes, then the outward appearance (of the coating) thereof may give a cloudy matted feeling, or may look whitish or feel rough, therefore tending to detract from the color (blackness, etc.) inherent in mascara; and a large quantity of powder could not be incorporated.

As an example of using a silicone-based powder, JP 11-12132 A (Patent Reference 3) describes a cosmetic for eyelashes with a silicone resin powder incorporated therein for the purpose of enhancing the uniformity of the finished film and for improving the feel in use thereof with no stickiness; and JP 5-43420 A (Patent Reference 4) discloses a cosmetic with a polymethyl-silsesquioxane powder and a hydrophobication-treated powder incorporated therein for the purpose of improving the feel thereof. However, the silicone-based powders described in these Patent References 3 and 4 both comprise spherical particles; and nothing is shown therein relating to use of a silicone-based powder of particles having such a spiky-surface-sugar-candy-ball-like shape that small protrusions are present over the surface thereof.

Patent Reference 1: JP 2003-246710 A
Patent Reference 2: JP 2004-315420 A
Patent Reference 3: JP 11-12132 A
Patent Reference 4: JP 5-43420 A

### DISCLOSURE OF THE INVENTION

### PROBLEMS THAT THE INVENTION IS TO SOLVE

The invention is directed to an object of providing a cosmetic for eyelashes which is excellent in the effect of imparting a feeling of voluminousness to eyelashes and which exerts high base transparency without damage to the color inherent in the cosmetic base.

### MEANS FOR SOLVING THE PROBLEMS

The present inventors have assiduously studied for the purpose of solving the above-mentioned problems and, as a result, have found that when a silicone-based powder having a specific morphology is incorporated in a cosmetic for eyelashes, then the above-mentioned problems can be solved, and have completed the present invention.

Specifically, the invention relates to a cosmetic for eyelashes which comprises (a) a silicone resin powder of particles having such a spiky-surface-sugar-candy-ball-like shape that small protrusions are present over the whole surface of each particle, (b) a volatile oil, and (c) an oily gelling agent.

The invention also relates to the above-mentioned cosmetic for eyelashes, further containing (d) a film-forming agent.

### EFFECTS OF THE INVENTION

According to the invention, there is provided an oil-based cosmetic for eyelashes which is excellent in the effect of imparting a feeling of voluminousness to eyelashes and which exerts high base transparency without damage to the color inherent in the cosmetic base.

### BEST MODE FOR CARRYING OUT THE INVENTION

The cosmetic for eyelashes of the invention is described in detail hereinunder.

As component (a) in the invention, used is a silicone resin powder of particles having such a spiky-surface-sugar-candy-ball-like shape that small protrusions are present over the whole surface of each particle. Not specifically defined, the silicone resin powder is preferably a polymethyl-silsesquioxane powder in view of its usability.

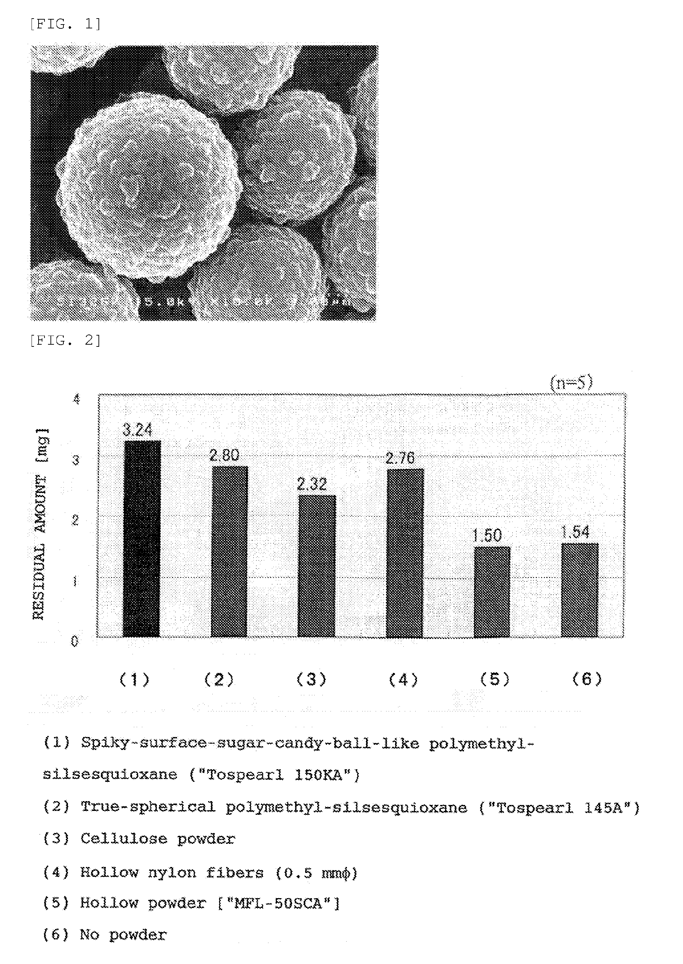

The silicone resin powder for use in the invention is characterized by its morphology in that the particles thereof have a pointed-sugar-candy-ball-like shape or a spiky-surface-sugar-candy-ball-like shape with small protrusions existing over the whole surface of each particle. Not specifically defined, the mean particle size is preferably from 3.5 to 6.5 µm or so. As the silicone resin powder of particles having such a spiky-surface-sugar-candy-ball-like shape, herein favorably used are, for example, a commercial product of "Tospearl 150KA" (by Momentive Performance Materials Inc.) and the like, to which, however, the invention is not limited. Fig. 1 shows an electromicroscopic photograph (SEM) of "Tospearl 150KA" powder particles.

Momentive sells "Tospearl 145A" or the like, which is a polymethyl-silsesquioxane powder of spherical particles with no small protrusions on the surface thereof. Comparing the above-mentioned "Tospearl 150KA" of spiky-surface-sugar-candy-ball-like particles with this "Tospearl 145A" of spherical particles in point of the oil absorption (where squalane was used as the oil) revealed that the oil absorption of the former was 61.3 (100 ml/mg) while that of the latter was 48.4 (100 ml/mg). The refractive index (n), the adhesion to eyelashes, the volume-boosting effect and others are shown in the section of Examples given hereinunder.

The amount of component (a) is preferably from 1 to 20% by mass of the total amount of the cosmetic, more preferably from 3 to 15% by mass, even more preferably from 5 to 10% by mass. When the amount is less than 1% by mass, then it is not enough for imparting the volume-boosting effect and therefore the cosmetic for eyelashes could hardly express the effect thereof. On the other hand, when the amount is more than 20% by mass, then the hardness of the cosmetic may increase too much, therefore detracting from the uniform application of the cosmetic to eyelashes.

As component (b), volatile oil (i.e., oil volatile at room temperature), preferably used is a low-boiling-point (having a boiling point of not higher than 260°C under normal pressure) isoparaffinic hydrocarbon oil, a low-boiling-point silicone oil or the like.

As the low-boiling-point isoparaffinic hydrocarbon oil (light isoparaffin), concretely, Isopar A, C, E, G, H, K, L and M (all by Exxon Mobil Corp.), Shellsol 71 (by Shell Chemicals Co.), Soltrol 100, 130 and 220 (all by Chevron Phillips Chemical Co.) and others are sold on the market and are commercially available.

Preferred examples of the low-boiling-point silicone oilinclude a linear silicone oil represented by the following formula (I), and a cyclic silicone oil (cyclomethicone) represented by the following formula (II).

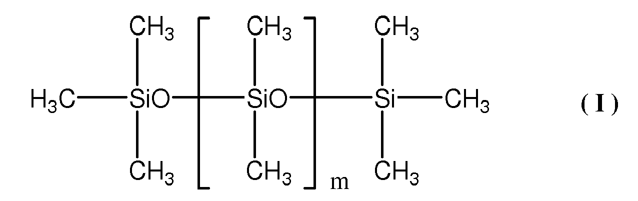

(In the formula, m indicates an integer of from 0 to 3.)

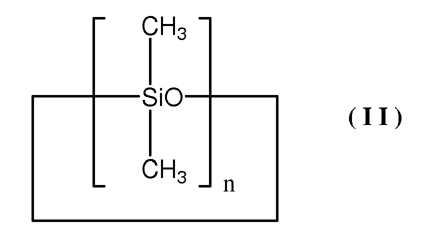

(In the formula, n indicates an integer of from 3 to 7.)
Preferred examples of the above low-boiling-point silicone oilinclude hexamethylcyclotrisiloxane, octamethyltetracyclosiloxane, decamethylcyclopentasiloxane, dodecamethylcyclohexasiloxane, and tetradecamethylcycloheptasiloxane.

As component (b) in the invention, for example, preferred is one comprising mainly a light isoparaffin such as "Isopar H" or the like, and this may be combined with a low-boiling-point silicone oil such as decamethylcyclopentasiloxane or the like. Preferably, the light isoparaffin and the low-boiling-point silicone oil are blended for use herein in a ratio of from 100/0 to 50/50 (by mass). One or more types may be used for component (b) .

The amount of component (b) is preferably from 10 to 80% by mass of the total amount of the cosmetic, more preferably from 20 to 70% by mass, even more preferably from 30 to 60% by mass. When the amount of component (b) is less than 10% by mass, then the component could not sufficiently exhibit the function as a solvent and is not enough for dissolving the other ingredients constituting the cosmetic, and therefore a uniform cosmetic enough for practical use could not be obtained. On the other hand, when the amount of component (b) is more than 80% by mass, the cosmetic could not have a suitable hardness but may be liquid, and it could not be uniformly applied to eyelashes, and in addition, the system stability could hardly be secured.

Examples of the oily gelling agent for component (c) include dextrin fatty acid esters, sucrose fatty acid esters, organic modified clay minerals, 12-hydroxystearic acid, and metal soaps, but not limited thereto.

The dextrin fatty acid ester is an ester of dextrin or reduced dextrin and a higher fatty acid. As the dextrin or reduced dextrin, preferably used here is one having a mean degree of glycopolymerization of from 3 to 100, more preferably from 10 to 50. As the higher fatty acid, preferably used here is a saturated fatty acid having from 8 to 24 carbon atoms, more preferably from 14 to 18 carbon atoms. Specific examples of the dextrin fatty acid ester include dextrin palmitate, dextrin palmitate/2-ethylhexanoate, dextrin stearate, dextrin palmitate/stearate, dextrin oleate, dextrin isopalmitate, and dextrin isostearate, and one or more of these may be used here. Commercial products are available, such as dextrin palmitate ("Rheopearl KL", "Rheopearl TL") and dextrin palmitate/2-ethylhexanoate ("Rheopearl TT") (all by Chiba Flour Milling Co., Ltd.), etc.

Examples of the sucrose fatty acid include sucrose stearate, sucrose acetate stearate, etc., concretely "Sugar Wax S-10E", "DK Ester S-160", "Sugar Wax A-10E" (all by Dai-ichi Kogyo Seiyaku Co., Ltd.).

The organic modified clay mineral includes clay minerals (e.g., montmorillonite, saponite, hectorite, bentonite, etc.) in which the exchangeable cation existing between the crystal layers has been substituted with an organic polar compound or an organic cation. Concretely, there are mentioned dimethyldistearylammonium hectorite (= quaternium-18 hectorite), dimethyldistearylammonim bentonite (= quaternium-18 bentonite), benzyldimethyldistearylammonium hectorite, dioctadecyldimethylammonium-modified montmorillonite, octadecyldimethylbenzylammonium-modified montmorillonite, and dihexadecyldimethylammoniummodified montmorillonite. Organic modified clay minerals for use in the invention are sold on the market, for example, as "Bentone 38" (= quaternium-18 hectorite), "Bentone 34" (= quaternium-18 bentonite", "Bentone 27" (= benzyldimethyldistearylammonium hectorite) (all by Rheox, Inc.), "Claytone 40", "Claytone SO" (all by Southern Clay Products, Inc.), etc., and are commercially available.

Apart from using such a previously-organic modified clay mineral, an unmodified clay mineral such as synthetic smectite (aluminium magnesium silicate) or the like and a cationic surfactant may be separately incorporated in a composition and the clay mineral may be organic-modified in the process of producing a gel composition.

Examples of the metal soap include aluminium isostearate, and calcium stearate.
One or more types may be used as component (c). The amount of component (c) is preferably from 1 to 25% by mass of the total amount of the cosmetic of the invention, more preferably from 3 to 20% by mass, even more preferably from 5 to 15% by mass. When the amount of component (c) is less than 1% by mass, then the cosmetic could not have a suitable hardness but may be liquid, and it could not be uniformly applied to eyelashes, and in addition, the system stability could hardly be secured. On the other hand, when component (c) is incorporated in an amount more than 25% by mass, then the hardness may be rather too high, and a uniform cosmetic enough for practical use could not be obtained.

In the invention, a film-forming agent (d) may be further incorporated in addition to the above components (a) to (c). Component (d), if incorporated, may enhance the resistance to sweat, tear, sebum and the like, may impart the effect of enhancing the curl retentiveness to eyelashes, and may improve the effect of preventing secondary adhesion (transfer) of the cosmetic to other sites (hair, skin). Not specifically defined, component (d) may be any resin that may be generally incorporated in cosmetics as a film-forming agent. Concretely, it includes latexes, such as polyvinyl alcohol, polyvinyl pyrrolidone, polyvinyl acetate, and polyalkyl acrylate; dextrin; cellulose derivatives such as alkyl cellulose and nitrocellulose; silicone resins such as trimethylsiloxysilicic acid; silicone-based resins, such as trimethylsiloxysilylpropylcarbamic acid, fluorine-modified silicone resin, and acryl-silicone copolymer resin; fluororesins, aromatic hydrocarbon resins, polymer emulsion resins, terpene resins, polybutene, polyisoprene, alkyd resins, polyvinylpyrrolidone-modified polymers, rosin-modified resins, and polyurethane. The fluororesin includes those having a pendant perfluoroalkyl group in the hydrocarbon main chain thereof, such as perfluoroalkyl group-having acrylic resins, perfluoroalkyl group-having methacrylic resins; those of which the main chain itself is a fluorocarbon such as polyvinylidene fluoride; those having both a hydrocarbon moiety and a fluorocarbon moiety in the main chain thereof formed through radical copolymerization of fluoroethylene and hydrocarbon vinyl ether, but not limited thereto.

Of those, especially preferred are silicone-type resins; and above all, herein usable are trimethylsiloxysilicic acid (commercially available as "KF7312J", and "X-21-5250" (both by Shin-etsu Silicones)) and tri(trimethylsiloxy)silylcarbamic acid (by Shin-etsu Silicones), to which, however, the invention should not be limited. One or more types may be used as component (d).

The amount of component (d), if incorporated, is preferably from 1 to 50% by mass of the total amount of the cosmetic of the invention, more preferably from 5 to 40% by mass, even more preferably from 10 to 30% by mass. When the amount is less than 1% by mass, then the cosmetic could not exhibit a sufficient curling effect and a curl-retaining effect, and is additionally poor in the effect of makeup-retaining potency of waterproofness, sweat resistance, sebum resistance. On the other hand, when the component is incorporated in an amount more than 50% by mass, then the hardness of the oily gel may tend to lower and the cosmetic could hardly be applied to eyelashes uniformly. Other disadvantages are that much time may be taken until drying, and eyelashes may tend to bunch together (with no separating effect) and their sticky feel may be significant.

In addition, as ingredients generally incorporated in cosmetics for eyelashes, an oil solid at room temperature, an oil liquid at room temperature (a fluid oil not evaporating at room temperature), a colorant and others may be suitably incorporated in the cosmetic for eyelashes of the invention.

Examples of the solid oil include paraffin, microcrystalline wax, polyethylene wax, candelilla wax, carnauba wax, bees wax, Japan tallow, jojoba ester, and synthetic wax. The ingredient, if incorporated, may enhance the temperature stability of the cosmetic for eyelashes, and may enhance the volume-boosting effect and the curing effect thereof. The amount of the solid oil, if any, is preferably from 1 to 20% by mass or so.

Examples of the liquid oil include hydrocarbon oils such as heavy isoparaffin, squalane, and liquid paraffin; esters, such as cetyl 2-ethylhexanoate, 2-ethylhexyl palmitate, 2-octyldodecyl myristate, neopentylglycol 2-ethylhexanoate, isopropyl myristate, and myristyl myristate; oils and fats, such as olive oil, avocado oil, jojoba oil, sunflower oil, safflower oil, camellia oil, macadamia nut oil, mink oil, liquid lanolin, lanolin acetate, and castor oil; silicone oils, such as dimethylpolysiloxane, methylphenylpolysiloxane, gum-like dimethylpolysiloxane having a high degree of polymerization, polyether-modified silicone, and amino-modified silicone; fluorooils, such as fluorine-modified dimethylpolysiloxane, fluorine-modified methylphenylpolysiloxane, perfluoropolyether, and perfluorocarbon. The ingredient, if incorporated, may improve the glossiness of the coating film after dried, and may increase the film strength. The amount of the liquid oil, if any, is preferably from 0.1 to 10% by mass or so.

Not specifically defined, the colorant may be any one generally usable in makeup cosmetics, but is preferably hydrophobic. For example, it includes inorganic pigments, such as talc, mica, kaolin, calcium carbonate, zinc flower, titanium dioxide, red iron oxide, yellow iron oxide, black iron oxide, ultramarine, carbon black, low-order titanium oxide, cobalt violet, chromium oxide, chromium hydroxide, cobalt titanate, bismuth oxychloride, and titanium/mica-base pearl pigment; organic pigments, for example, zirconium, barium or aluminium lakes, such as Red No. 201, Red No. 202, Red No. 204, Red No. 205, Red No. 220, Red No. 226, Red No. 228, Red No. 405, Orange No. 203, Yellow No. 205, Yellow No. 4, Yellow No. 5, blue No. 1, Blue No. 404, and Green No. 3; natural colorants such as chlorophyll, and (β-carotene; resin powders, such as nylon, cellulose, and polyethylene; and dyes. The amount of the colorant is preferably from 0.1 to 30% by mass or so.

Preferably, the cosmetic for eyelashes of the invention is solid, pasty or liquid within a range within which it may have a viscosity to such a degree that a powder or a pigment could be stably dispersed therein.

Further in accordance with the intended use, any other ingredients that may be generally in makeup cosmetics may be added to the cosmetic for eyelashes of the invention within a quantitative and qualitative range not detracting from the effect of the invention. The ingredients include fibers, alcohols, polyalcohols, chemicals, surfactants, polymers, preservative, fragrance, antioxidant, UV absorbent, moisturizing agent, and water, but not limited thereto.

### EXAMPLES

The invention is described in more detail with reference to the following Examples, by which, however, the invention should not be restricted at all. Unless otherwise specifically indicated, the amount added is expressed as % by mass relative to the system in which the ingredient is incorporated.

### (Example 1)

Various types of powders were incorporated and mixed with component (b) serving as the main solvent of the cosmetic for eyelashes of the invention, and the samples were tested for the transparency and the properties thereof.

Concretely, light isoparaffin ("Isopar H" by Exxon Mobile Corp.) and a powder shown in Table 1 below were mixed in a ratio of light isoparaffin/powder = 2/1 (by mass), and the mixture was visually checked for the behavior thereof. The transparency was evaluated according to the standards mentioned below. The results are shown in Table 1. (Standards for Transparency Evaluation)
Excellent: Highly transparent.
Average: Semitransparent.
Not Good: Poorly transparent.

**[Table 1]**

| Powder | Properties | Transparency |
|---|---|---|
| Silica ("Spherical Silica P1500", by JGC Catalysts and Chemicals Ltd.) | liquid | average |
| Silica ("Sunsphere L-51 ", by Asahi Glass Co.) | gel | average |
| (Vinyldimethicone/methicone-silsesquioxane) cross polymer | paste | not good |
| (Diphenyldimethicone/vinyldiphenyldimethicone/silsesquioxane) cross polymer | emulsion | not good |
| Methyl methacrylate cross polymer | liquid | not good |
| HDL/trimethylolhexyl-lactone cross polymer | liquid | not good |
| Cellulose | liquid | not good |
| (Dimethicone/vinyldimethicone) cross polymer | emulsion | not good |
| Polymethyl methacrylate | liquid | not good |
| Nylon-12 | liquid | not good |
| Polymethyl-silsesquioxane ("Tospearl 145A", true-spherical) | liquid | average |
| Polymethyl-silsesquioxane ("Tospearl 150KA", spiky-surface-sugar-candy-ball-like shape) | liquid | excellent |

As is obvious from the results in Table 1, only the sample comprising, as the powder, the polymethyl-silsesquioxane ("Tospearl 150KA") having a spiky-surface-sugar-candy-ball-like shape which is component (a) in the invention gave a uniform dispersion in the solvent of light isoparaffin, of which the appearance was not cloudy and was excellent in transparency.

### (Example 2)

A powder of true-spherical polymethyl-silsesquioxane ("Tospearl 145A" made by Momentive Performance Material Inc.), and a powder having a spiky-surface-sugar-candy-ball-like shape polymethylsilsesquioxane ("Tospearl 150KA" made by Momentive Performance Material Inc.) for use in the invention were analyzed for the refractive index thereof.

Concretely, 2 g of the powder was dispersed in 10 g of light isoparaffin ("Isopar H" by Exxon Mobile Corp., having a refractive index (n) = 1.423), and a low-boiling-point silicone oil, decamethylcyclopentasiloxane (refractive index (n) = 1.398) was added thereto dropwise in an amount of 0.1 g each. The refractive index of the mixed oil that had become most transparent was measured, and the value was taken as the refractive index of the powder.

As a result, the refractive index (n) of the true-spherical polymethyl-silsesquioxane was 1.410: and the refractive index (n) of the polymethyl-silsesquioxane having a spiky-surface-sugar-candy-ball-like shape was 1.419.

Specifically, the refractive index of component (a) in the invention was extremely approximate to the refractive index of the light isoparaffin that is the main solvent of mascara. Accordingly, use of the silicone resin powder of particles having a spiky-surface-sugar-candy-ball-like shape provides a cosmetic for eyelashes having an extremely high cosmetic base transparency.

### (Example 3)

A powder of true-spherical polymethyl-silsesquioxane ("Tospearl 145A" by Momentive Performance materials Inc.), and a powder of polymethyl-silsesquioxane having a spiky-surface-sugar-candy-ball-like shape ("Tospearl 150KA" by Momentive Performance materials Inc.) for use in the invention were analyzed for the refractive index thereof were compared in point of the adhesiveness thereof to eyelashes according to the adhesion comparison test method mentioned below.

As a coating sample, prepared was a dispersion of powder/light isoparaffin ("Isopar H") = 1/1 (by mass).

As a sample of eyelashes, hairs (strands) were banded into bundles of about 30 hairs each; and the bundles were cut to have a length of 2.5 cm. These were tested.

### (Test Method)

A suitable amount of the coating sample was taken with a mascara brush, and applied three times to the sample of eyelashes, and dried at 37°C for 15 minutes. From the mass of the hair sample before and after the coating sample application thereto, the adhered amount of the coating sample was computed. As a result, the adhered amount of the coating sample containing the true-spherical polymethyl-silsesquioxane ("Tospearl 145A") was 76.3 mg, while that of the coating sample containing the polymethyl-silsesquioxane having a spiky-surface-sugar-candy-ball-like shape ("Tospearl 150KA") for use in the invention was 147.0 mg; or that is, the adhered amount of the latter was dramatically higher than that of the former.

### (Example 4)

Mascara samples having the composition mentioned below and containing any of the powders (1) to (5) mentioned below in an amount of 5% by mass were prepared. Each mascara sample was applied three times to a sample of eyelashes [prepared by banding hairs (strands) into bundles of about 30 hairs each followed by cutting them to have a length of 2.5 cm], and dried at 37°C for 30 minutes. The difference in the mass of the hair sample before and after the mascara sample application thereto was computed, and this is the residual mascara sample amount. As a control, a sample not containing the powder was tested in the same manner. The results are shown in Fig. 2.

### (Tested Powder)

(1) Polymethyl-silsesquioxane having a spiky-surface-sugar-candy-ball-like shape ("Tospearl 150KA"),
(2) True-spherical polymethyl-silsesquioxane ("Tospearl 145A"),
(3) Cellulose powder,
(4) Hollow nylon fibers (0.5 mmφ) having a high volume-boosting effect,
(5) Hollow powder ["MFL-50SCA" (by Matsumoto Yushi-Seiyaku Co., Ltd.), used in Patent reference 2 (= JP 2004-315420 A)].

| [Mascara] | |
|---|---|
| Light isoparaffin ("Isopar H") | balance |
| Powder (of the above (1) to (5)) | 5.0 |
| Microcrystalline wax | 3.0 |
| Polyethylene | 5.0 |
| Sucrose fatty acid ester | 5.0 |
| Dextrin fatty acid ester | 5.0 |
| Quaternium-18 hectorite | 10.0 |
| Polyether-modified silicone surfactant | 2.0 |
| POE triisostearate hardened castor oil (20 E.O.) | 2.0 |
| Trimethylsiloxysilicic acid | 15.0 |
| Macadamia nut oil | 0.1 |
| Hydrophobicated colorant (black) | 8.0 |
| Pearling agent | 1.0 |
| Ion-exchanged water | 2.0 |
| 1,3-Butylene glycol | 2.0 |

As is obvious from the results shown in Fig. 2, the residual amount in the case where polymethyl-silsesquioxane having a spiky-surface-sugar-candy-ball-like shape that is component (a) in the invention was used as the powder was the largest, therefore suggesting the excellent volume-boosting effect of this case.

### (Example 5)

Of the mascara samples prepared in Example 4, the sample with no powder (= control sample), the sample with 5% by mass of "Tospearl 150KA", the sample with 5% by mass of hollow nylon fibers (0.5 mmφ) having a high volume-boosting effect, and the sample with 5% by mass of hollow powder ("MFL-50SCA") were separately filled in transparent enamel bottles, and the blackness of their appearance was evaluated in point of the brightness thereof and by organoleptic evaluation.

The brightness (L value) was measured, suing a spectrophotometer system of Macbeth COLOR-EYE 7000. Samples having a lower brightness (L value) do not detract from the blackness of the cosmetic base.

The organoleptic evaluation was based on the following standards:

### (Evaluation Standards)

Excellent: The blackness of the appearance was on the same level as that of the control.
Good: The blackness of the appearance was somewhat lower than that of the control.
Average: The blackness of the appearance was lower than that of the control, and the appearance became somewhat grayish.
Not good: As compared with that of the control, the appearance was grayish.
The results are shown in Table 2.

**[Table 2]**

| Powder | L Value | Organoleptic Evaluation |
|---|---|---|
| Polymethyl-silsesquioxane ("Tospearl 150A", having a spiky-suraface-sugar-candy-ball-like shape) | 25.77 | excellent |
| Hollow nylon fibers (0.5 mmφ) | 26.71 | good |
| Hollow powder ("MFL-50SCA") | 28.09 | average |
| No powder (= control sample) | 25.67 | control |

As is obvious from the results shown in Table 2, the brightness of the mascara with "Tospearl 150KA" incorporated therein was on the same level as that of the sample with no powder, and this confirms that the powder "does not detract from the blackness" of mascara. In addition, as compared with the mascara with a hollow powder ("MFL-50SCA"), which was incorporated therein for the purpose of satisfying both the volume-boosting effect and the curling effect in Patent Reference 2 (= JP 2004-315420 A), it was confirmed that the powder "Tospearl 150KA" did not whiten the mascara base. The mascara with hollow nylon fibers for enhancing the feeling of voluminousness was not good in the organoleptic evaluation.

### (Example 5)

Of the mascara samples prepared in Example 4, the mascara of the invention with "Tospearl 150KA" incorporated therein was stored at different temperatures of -20°C, -5°C, 0°C, room temperature (25°C), 37°C and 50°C for 4 weeks. After thus stored, the samples were checked for the color and the appearance thereof, and compared with the samples just after their preparation (three lots were tested). As a result, there was found little change in the appearance and the color between the stored samples and the fresh samples, and it confirms excellent temperature stability of the samples.

Other formulation examples are shown below.

### (Example 6)

| (Constitutive Ingredients) | (% by mass) |
|---|---|
| Light isoparaffin ("Isopar H") | balance |
| Polymethyl-silsesquioxane ("Tospearl 150KA") | 5.0 |
| Polyethylene | 18.0 |
| Dextrin fatty acid ester | 11.0 |
| Triethylhexanoin | 1.0 |
| Isostearic acid | 2.0 |
| Trimethylsiloxysilicic acid | 30.0 |
| Hydrophobicated colorant (black) | 5.0 |
| Dibutylhydroxytoluene | 0.05 |

### (Example 7)

| (Constitutive Ingredients) | (% by mass) |
|---|---|
| Light isoparaffin ("Isopar H") | balance |
| Cyclomethicone | 10.0 |
| Polymethyl-silsesquioxane ("Tospearl 150KA") | 10.0 |
| Candelilla wax | 3.0 |
| Bees wax | 3.0 |
| Microcrystalline wax | 1.0 |
| Dextrin fatty acid ester | 9.0 |
| Quaternium-18 hectorite | 3.0 |
| Triethylhexanoin | 2.0 |
| Isostearic acid | 1.0 |
| Trimethylsiloxysilicic acid | 10.0 |
| Fluorine-modified silicone resin | 5.0 |
| Hydrophobicated colorant (black) | 7.0 |
| Dibutylhydroxytoluene | 0.05 |

### (Example 8)

| (Constitutive Ingredients) | (% by mass) |
|---|---|
| Light isoparaffin ("Isopar H") | balance |
| Polymethyl-silsesquioxane ("Tospearl 150KA") | 20.0 |
| Microcrystalline wax | 3.0 |
| Polyethylene | 5.0 |
| Sucrose fatty acid ester | 1.0 |
| Dextrin fatty acid ester | 10.0 |
| Quaternium-18 hectorite | 3.0 |
| Polyether-modified silicone surfactant | 2.0 |
| POE triisostearate hardened castor oil (20 E.O.) | 2.0 |
| Trimethylsiloxysilicic acid | 7.0 |
| Hydrophobicated colorant (black) | 5.0 |
| Ion-exchanged water | 3.0 |
| 1,3-Butylene glycol | 1.0 |

### BRIEF DESCRIPTION OF THE DRAWINGS

[Fig. 1] This is an electromicroscopic photograph (SEM) showing the morphology of silicone resin powder particles having a spiky-surface-sugar-candy-ball-like shape for use in the invention.
[Fig. 2] This is a graph showing the test results of the mascara residual amount in Example 4.

### INDUSTRIAL APPLICABILITY

The cosmetic for eyelashes of the invention is excellent in the effect of imparting a feeling of voluminousness to eyelashes and exerts an excellent effect of high base transparency without damage to the color inherent in the cosmetic base.

## Claims

1. A cosmetic for eyelashes which comprises (a) a silicone resin powder of particles having such a spiky-surface-sugar-candy-ball-like shape that small protrusions are present over the whole surface of each particle, (b) a volatile oil, and (c) an oily gelling agent.

2. The cosmetic for eyelashes as claimed in claim 1, wherein component (b) is one or more selected from a low-boiling-point isoparaffinic hydrocarbon oil and a low-boiling-point silicone oil.

3. The cosmetic for eyelashes as claimed in claim 1 or 2, containing from 1 to 20% by mass of component (a), from 10 to 80% by mass of component (b) and from 1 to 25% by mass of component (c).

4. The cosmetic for eyelashes as claimed in claim 1, further containing (d) a film-forming agent.

5. The cosmetic for eyelashes as claimed in claim 4, containing from 1 to 50% by mass of component (d).
